# EUROPÄISCHE PATENTSCHRIFT

(11) **EP 1 246 786 B1**
(45) Veröffentlichungstag und Bekanntmachung des Hinweises auf die Patenterteilung: **15.06.2005**
(21) Anmeldenummer: 00987470.2
(22) Anmeldetag: 28.12.2000
(51) Int. Cl.: C07C 29/90, C07C 29/80, C07C 31/22

(54) **FARBZAHLVERBESSERUNG VON MEHRWERTIGEN ALKOHOLEN DURCH HYDRIERUNG**
COLOUR NUMBER IMPROVEMENT IN POLYHYDRIC ALCOHOLS BY HYDROGENATION
AMELIORATION DE L'INDICE DE COULEUR D'ALCOOLS POLYVALENTS PAR HYDROGENATION

(30) Priorität: 28.12.1999 DE 19963442
(43) Veröffentlichungstag der Anmeldung: 09.10.2002
(73) Patentinhaber: BASF AKTIENGESELLSCHAFT, 67056 Ludwigshafen (DE)
(72) Erfinder: DERNBACH, Matthias, 69221 Dossenheim (DE); KRATZ, Detlef, 69121 Heidelberg (DE); Dr. STAMMER, Achim, Mobile, Alabama 36695 (US); HAAKE, Mathias, 68161 Mannheim (DE); KOCH, Michael, 67346 Speyer (DE); SCHULZ, Gerhard, 67098 Bad Dürkheim (DE)
(74) Vertreter: Isenbruck, Günter, Dr.
(86) Internationale Anmeldenummer: PCT/EP2000/013328
(87) Internationale Veröffentlichungsnummer: WO 2001/047850

(56) Entgegenhaltungen:
- EP-A- 0 601 571
- WO-A-98/28253
- SU-A- 125 552
- CHEMICAL ABSTRACTS, vol. 55, no. 21, 16. Oktober 1961 (1961-10-16) Columbus, Ohio, US; abstract no. 20996h, Spalte 20996; XP002171005 -& NL 97 091 C (STAMICARBON) 15. Februar 1961 (1961-02-15)

## Beschreibung

Die vorliegende Erfindung bezieht sich auf ein Verfahren, bei dem mehrwertige Alkohole geringer Farbzahl durch Hydrierung erhalten werden.

Mehrwertige Alkohole werden in großem Maßstab durch Kondensation von Formaldehyd mit höheren, CH-aciden Aldehyden oder mit Wasser und Acrolein bzw. 2-Alkylacroleinen erhalten. Dabei unterscheidet man bei dieser Reaktion zwischen zwei prinzipiellen Durchführungsvarianten.

Zum einen ist dies das sogenannte Cannizzaro-Verfahren, das wiederum unterteilt wird in das anorganische und das organische Cannizzaro-Verfahren. Bei der anorganischen Variante setzt man einen Überschuß an Formaldehyd mit dem entsprechenden Alkanal in Gegenwart von stöchiometrischen Mengen einer anorganischen Base wie NaOH oder Ca(OH)₂ um. Das in der ersten Stufe gebildete Dimethylolbutanal reagiert in der zweiten Stufe mit dem überschüssigen Formaldehyd in einer Disproportionierungsreaktion zu Trimethylolpropan und dem Formiat der entsprechenden Base, also etwa zu Natrium- oder Kalziumformiat. Der Anfall dieser Salze stellte einen Nachteil dar, da sie schwierig vom Reaktionsprodukt abzutrennen sind, und außerdem ein Äquivalent Formaldehyd verloren geht.

Bei dem organischen Cannizzaro-Verfahren wird anstelle einer anorganischen Base ein tertiäres Alkylamin eingesetzt. Damit lassen sich höhere Ausbeuten erzielen als mit einer anorganischen Base. Es fällt als unerwünschtes Nebenprodukt Trialkylammoniumformiat an. Somit geht auch hier ein Äquivalent des Formaldehyds verloren.

Die Nachteile des Cannizzaro-Verfahrens werden bei dem sogenannten Hydrierverfahren vermieden. Dabei bringt man Formaldehyd mit dem entsprechenden Aldehyd in Gegenwart von katalytischen Mengen eines Amins zur Reaktion. Damit wird erreicht, daß die Reaktion auf der Stufe des alkylolierten Aldehyds anhält. Nach Abtrennung des Formaldehyds wird das Reaktionsgemisch, das neben dem erwähnten alkylolierten Aldehyd noch geringe Mengen des entsprechenden mehrwertigen Alkohols und von Acetalen der gebildeten Alkohole enthält, einer Hydrierung unterworfen, bei der der gewünschte mehrwertige Alkohol erhalten wird.

Ein besonders effektives Verfahren zur Herstellung von durch Kondensation von Aldehyden mit Formaldehyd erhältlichen Alkoholen wird dabei in der WO 98/28253 beschrieben. Hohe Ausbeuten, verbunden mit den Anfallen geringer Mengen an Koppelprodukten, werden mit diesem Verfahren ermöglicht. Es wird dabei so verfahren, daß der höhere Aldehyd mit der 2- bis 8-fachen Menge Formaldehyd in Gegenwart eines tertiären Amins umgesetzt wird, und man das so erhaltene Reaktionsgemisch in zwei Lösungen auftrennt, wobei eine Lösung das erwähnte vollständig methylolierte Alkanal und die andere Lösung nicht umgesetztes Ausgangsprodukt aufweist. Diese letzte Lösung wird in die Reaktion zurückgeführt. Die Auftrennung erfolgt durch Destillation oder einfaches Abtrennen der wässrigen von der organischen Phase. Die das Produkt enthaltende Lösung wird einer katalytischen und/oder thermischen Behandlung unterworfen, um nicht-vollständig alkylolierte Alkanale in die gewünschten vollständig methyloliertcn Verbindungen zu überführen. Hierbei entstandenes Nebenprodukt wird durch Destillation abgetrennt, und der so erhaltene Sumpf wird der katalytischen Hydrierung, die zu den mehrwertigen Alkoholen führt, unterworfen.

Beispiele für wichtige, mit den beschriebenen Verfahren hergestellte Alkohole sind Neopentylgylkol, Pentaerythrit, Trimethylolethan, Trimethylolbutan und insbesondere Trimethylolpropan (TMP).

TMP hat ein breites Einsatzfeld als Vemetzer von Polyestern und von Polyurethanen gefunden. Kommerziell erhältliche TMP-Qualitäten weisen jedoch eine mehr oder weniger ausgeprägte Färbung auf, die vermutlich durch das Vorliegen von Verunreinigungen verursacht wird. Bei vielen Verwendungen ist diese Färbung nicht störend. Es gibt jedoch auch Anwendungen, in denen der Einsatz von möglichst farblosem TMP wünschenswert ist. In der Literatur sind verschiedene Verfahren beschrieben, mit denen eine Farbzahlverbesserung des TMP erreicht werden soll.

Die US 3,097,245 beschreibt ein Verfahren zur Herstellung von Trimethylolpropan mit einer APHA-Farbzahl zwischen 50 und 200. Diese Farbzahl wird dabei durch das Einhalten bestimmter Reaktionsbedingungen hinsichtlich Temperatur, Reaktionszeit, pH-Wert und Konzentration der Ausgangsverbindungen erreicht. Weiterhin schließt sich an die Reaktion die Behandlung der erhaltenen Lösung mit einem Ionenaustauscherharz an.

Die US 5,603,835 offenbart ein Verfahren zur Herstellung von TMP mit APHA-Farbzahlen von < 100. Diese werden erreicht durch extraktive Nachbehandlung der erhaltenen rohen TMP-Lösungen mit einem Ether oder einem Ester. Die eingesetzten TMP-Lösungen stammen im allgemeinen aus dem Cannizzaro-Verfahren.

Die beiden vorstehend beschriebenen Verfahren weisen den Nachteil auf, daß sie relativ aufwendig sind, da bestimmte Bedingungen genau eingehalten werden müssen und die Zugabe eines Ionenaustauscherharzes nötig ist, bzw. die Einrührung mindestens eines Lösungsmittels notwendig wird.

Zur Hydrierung von bei der Kondensation von Formaldehyd mit höheren Aldehyden entstehenden Produkten finden sich in der Literatur nur wenige Informationen.

Die DE-A-17 68 259 offenbart ein Verfahren zur Verarbeitung der Nebenprodukte, die bei der Umsetzung von Formaldehyd mit höheren Aldehyden zu mehrwertigen Alkoholen entstehen. Das Verfahren besteht darin, diese Nebenprodukte nach der Abtrennung vom Hauptprodukt einer Hydrierung zu unterwerfen. Dabei werden in größeren Mengen hauptsächlich aliphatische Alkohole gewonnen.

Die EP-A 0 601 571 beschreibt ein Verfahren zur Reinigung von 1,4-Butandiol, das durch Hydrolyse von Diacetoxybutan erhalten wurde. Dabei wird von dem Hydrolysat in einer ersten Destillation Wasser und Essigsäure als Kopfprodukt abgetrennt, das Sumpfprodukt wird einer zweiten Destillation unterworfen, in der Acetoxybutan- und Hydroxyacetoxybutan abgetrennt werden. Anschließend wird das rohe 1,4-Butandiol einer Hydrierung unterworfen, an die sich eine weitere Destillation anschließt.

Die NL-C 97 091 offenbart ein Verfahren zur Reinigung von Cyclohexanol, das durch Oxidation von Cyclohexan und Verseifung des entstehenden Esters erhalten wurde. Das Cyclohexanol wird dabei einer Hydrierung unterworfen und anschließend destilliert.

Das in der SU-A 125 552 beschriebene Hydrierverfahren dient der Reinigung von nach dem Cannizzaro-Verfahren erhaltenem TMP. Es wird dabei entweder rohes TMP in Form einer wässrigen Lösung enthaltend ca. 30 % TMP, oder ein gereinigtes, von Wasser und Formiaten befreites TMP mit einem Gehalt von ca. 80 % an TMP eingesetzt. Man erhält durch Hydrierung an Nickel-, Zink-, Molybdän- und Kupferkatalysatoren reines TMP mit einem Gehalt von ca. 98 % nach Destillation. Die verwendeten Drücke betragen 1 bis 250 bar, vorzugsweise 10 bis 200 bar, die Temperaturen sind 20 bis 200°C, vorzugsweise 100 bis 150°C. Es wird erwähnt, dass das erhaltene TMP farblos ist, wobei jedoch keine Farbzahl angegeben wird.

Es hat sich jedoch gezeigt, dass die mit diesem Verfahren erhältlichen Farbzahlverbesserungen für viele Zwecke oft nicht ausreichend sind.

Die der vorliegenden Erfindung zugrunde liegende Aufgabe ist daher die Bereitstellung eines Verfahrens, das es erlaubt, mehrwertige Alkohole, insbesondere TMP, mit geringer Farbzahl zu erhalten. Dabei sollte es möglich sein, APHA-Farbzahlen von < 20 zu erreichen.

Diese Aufgabe wird gelöst durch ein Verfahren zur Farbzahlverbesserung von mehrwertigen Alkoholen durch katalytische Hydrierung, dadurch gekennzeichnet, dass in der Hydrierung ein mehrwertiger Alkohol eingesetzt wird, der nach seiner Herstellung durch Destillation gereinigt wurde.

Das erfindungsgemäße Verfahren kann dabei zur Farbzahlverbesserung von mehrwertigen Alkoholen, insbesondere TMP, jeglicher Herkunft verwendet werden. Chargen, die dem organischen oder dem anorganischen Cannizarro-Verfahren entstammen, können ebenso in der Farbzahlverbesserungshydrierung nach der vorliegenden Erfindung eingesetzt werden wie aus dem Hydrierverfahren stammende Alkohole. Es ist hierbei jedoch wichtig, dass der Alkohol vorher gereinigt wurde und eine Reinheit aufweist, die in einem geeigneten Bereich liegt und eine Verbesserung der Farbzahl mittels des erfindungsgemäßen Verfahrens gestattet. Es wurden besonders gute Ergebnisse erzielt, wenn der eingesetzte mehrwertige Alkohol dem Hydrierverfahren entstammte. Der Einsatz von mehrwertigen Alkoholen, insbesondere TMP, dieser Herkunft ist sonst erfindungsgemäß bevorzugt.

Es wurde festgestellt, daß durch den Einsatz von bereits destilliertem mehrwertigem Alkohol eine Farbzahlverbesserung erreicht werden kann, die weitaus größer ist als bei einem nicht destillativ vorgereinigten Alkohol. Gute Ergebnisse wurden dabei mit Lösungen erhalten, die einen Alkohol-Gehalt von > 95 % aufweisen.

Wenn TMP in die Hydrierung eingesetzt wird, können besonders gute Ergebnisse beim Einsatz von TMP-Lösungen mit einem Gehalt von > 98 % erzielt werden.

Die erfindungsgemäße Hydrierung ist insbesondere auf alle mehrwertigen Alkohole anwendbar, die durch Kondensation von Formaldehyd mit höheren Aldehyden unter Zugabe katalytischer Mengen Trialkylamin und nachfolgender Hydrierung hergestellt werden können. Geeignete höhere Aldehyde sind praktisch alle Alkanale mit einem aciden Wasserstoffatom in α-Stellung zur Carbonylgruppe. Es können aliphatische Aldehyde mit 2 bis 24 C-Atomen als Ausgangsmaterialien verwendet werden, die geradkettig oder verzweigt sein oder auch alicyclische Gruppen enthalten können. Ebenso sind araliphatische Aldehyde als Ausgangsstoffe geeignet, vorausgesetzt daß sie eine Methylengruppen in α-Stellung zur Carbonylgruppe enthalten. Im allgemeinen werden Aralkylaldehyde mit 8 bis 24 C-Atomen, vorzugsweise mit 8 bis 12 C-Atomen als Ausgangsmaterialien verwendet, beispielsweise Phenylacetaldehyd. Bevorzugt werden aliphatische Aldehyde mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende n-pentanale, -n-hexanale,-n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methyl-hexanal, 3-Methylheptanal; 4-Methyl-pentanal, 4-Methyl-heptanal, 5-Methyl-hexanal, 5-Methylheptanal; 3,3,5-Trimethyl-n-pentyl-, 3,3-Diethylpentyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4,-Tetramethylpentylaldehyd; insbesondere C₂ bis C₁₂-n-Alkanale.

Besonders bevorzugte mehrwertige Alkohole im Rahmen der vorliegenden Erfindung sind Trimethylolethan, Trimethylolpropan, Trimethylolbutan, Neopentylglykol und Pentaerythrit. Der meist bevorzugte Alkohol ist Trimethylolpropan.

Soll mit dem erfindungsgemäßen Verfahren die Farbzahl von TMP verbessert werden, kann ein nach dem Hydrierverfahren hergestelltes TMP hoher Reinheit (> 98 %) beispielsweise erhalten werden nach dem in der deutschen Anmeldung mit dem Titel "Verfahren zur Reinigung von durch Hydrierung hergestelltem Trimethylolpropan durch kontinuierliche Destillation", Aktenzeichen 199 63 435.1 (Anmelder: BASF AG) beschriebenen Verfahren. Dabei wird das nach Hydrierung erhaltene Rohprodukt zunächst einer Entwässerung unterzogen, bei der Wasser und andere Leichtsieder wie Methanol, Trialkylamin oder Trialkylammoniumformiat durch Destillation abgetrennt werden. Diese Destillation kann dabei bei Drücken< 400 mbar, vorzugsweise 20 bis 200 mbar, Sumpftemperaturen < 200°C und kurzen Verweilzeiten so durchgeführt werden, daß das gebildete Trialkylammoniumformiat mit TMP nur in geringem Maß zu TMP-Formiaten und Trialkylamin abreagiert. Es ist auch möglich, die Destillation bei Drück von > 200 mbar, vorzugsweise > 400 mbar, Sumpftemperaturen von > 140°C und langen Verweilzeiten so durchzuführen, daß zumindest der überwiegende Teil des TMP mit Trialkylammoniumformiat zu TMP-Formiaten und Trialkylamin reagiert.

Anschließend werden im nächsten Schritt die Hochsieder abgetrennt. Dies geschieht dadurch, daß vom Sumpf bei 210 bis 250°C diejenigen Komponenten durch Destillation abgetrennt werden, die bei diesen Temperaturen flüchtig sind. Die Hochsieder verbleiben somit im Sumpf. Die erhaltene, leichtsiedende TMP-reiche Fraktion wird anschließend destillativ aufgearbeitet (erste Reindestillation), wobei unerwünschte Leichtsieder abgetrennt werden. Das erhaltene Reinprodukt kann einer zweiten Reindestillation unterworfen werden, um ein besonders sauberes Produkt zu erhalten.

Der Inhalt der erwähnten deutschen Anmeldung ist ein wichtiger und integraler Teil der vorliegenden Erfindung und durch Referenz in die vorliegende Anmeldung eingeschlossen.

Das in dieser Anmeldung beschriebene Verfahren kann auch noch weiter variiert werden. So ist es beispielsweise möglich, gebildetes TMP-Formiat mit einem geeigneten Amin, vorzugsweise einem Dialkylamin, zu TMP und Dialkylformamid umzusetzen. Ein solches Verfahren ist in der deutschen Anmeldung mit dem Titel "Verfahren zur Umwandlung von bei der Trimethylolalkan-Herstellung gebildeten Trimethylolalkanformiat", Aktenzeichen 199 63 444.0 (Anmelderin: BASF AG) beschrieben.

Weiterhin kann auch eine Steigerung der Ausbeute durch Zersetzung der Hochsieder mittels Säurezugabe zu TMP und anderen Produkten erzielt werden. Die Beschreibung eines derartigen Verfahrens findet sich in der deutschen Anmeldung mit dem Titel "Verfahren zur Zersetzung von bei der Synthese mehrwertiger Alkohole gebildeter Nebenprodukte", Aktenzeichen 199 63 437.8 (Anmelderin: BASF AG).

Es konnten gute Ergebnisse mit einem derart oder auf eine andere Weise destillativ gereinigten TMP, das APHA-Farbzahlen von 10 bis 500 APHA, vorzugsweise 20 bis 120 APHA aufweist, erhalten werden.

Durch die Hydrierung nach der vorliegenden Erfindung lassen sich mehrwertige Alkohole mit APHA-Farbzahlen < 10 herstellen. Insbesondere läßt sich TMP mit Farbzahlen von bis zu ≤ 6 APHA erhalten.

Die erfindungsgemäße Hydrierung wird bei Temperaturen von 20 bis 300°C, vorzugsweise 100 bis 180°C durchgeführt, wobei Drücke von 1 bis 350 bar, vorzugsweise 1 bis 100 bar, Anwendung finden. Die dabei benutzten Verweilzeiten betragen 5 Minuten bis 4 Stunden, vorzugsweise 10 Minuten bis 1 Stunde. Es kann dabei eine diskontinuierliche, sogenannte Batch-Fahrweise gewählt werden, die vorzugsweise im Rührkessel durchgeführt wird. Ebenso gut ist es möglich, die Hydrierung kontinuierlich durchzuführen, vorzugsweise in Rohrreaktoren in der Sumpf- oder Rieselfahrweise.

Als Katalysatoren finden in dem erfindungsgemäßen Verfahren heterogene Katalysatoren Verwendung, die generell in Hydrierungen benutzt werden. Solche Katalysatoren sind einem Fachmann bekannt. Sie enthalten generell Metalle der 3. bis 12. Gruppe des Periodensystems, beispielsweise Ruthenium, Osmium, Iridium, Mangan, Platin, Palladium, Rhodium, Molybdän, Wolfram, Chrom, Eisen, Kobalt, Nickel, Vanadin und Zink sowie Kombinationen dieser Metalle, die sowohl in Form der reinen Metalle als auch von deren Verbindungen, beispielsweise Oxyden oder Sulfiden, eingesetzt werden können. Vorzugsweise werden Kupfer-, Nickel-, Ruthenium- oder Palladiumkatalysatoren verwendet. Diese Katalysatoren können auf den üblichen Trägern, beispielsweise Al₂O₃, SiO₂, TiO₂ oder Kohlefasem aufgebracht sein. Die so erhaltenen, geträgerten Katalysatoren können in allen bekannten Konfektionierungsformen vorliegen. Beispiele sind Stränge oder Tabletten.

Bevorzugt ist der Einsatz von Dünnschichtkatalysatoren der oben erwähnten Metalle, bei denen die Aktivkomponente auf ein geeignetes Gewebe aufgebracht wurde. Geeignete Gewebematerialien sind organische oder anorganische Materialien oder Metalle, die beispielsweise in Form von Gestricken oder Geweben verwendet wurden. Beispiele für geeignete Materialien finden sich in der EP-A-627 944 und der EP-A-564 830. Beispiele für bevorzugte Metalle sind Edelstahl oder Kanthal. Das Aufbringen der Aktivkomponente kann nach den üblichen, einem Fachmann bekannten Verfahren erfolgen, beispielsweise durch Aufdampfen oder nach dem Tränkverfahren.

Gemäß einer bevorzugten Ausführungsform der vorliegenden Erfindung werden Raney-Nickel-Dünnschichtkatalysatoren verwendet, die durch alternierendes Aufdampfen in Vakuum von Aluminium und Nickel auf geeignete Träger, vorzugsweise Edelstahlträger, erhalten werden und die Katalysator-Schichtdicken bis ca. 100 µm aufweisen. Alternativ kann auch eine vorgefertigte Raney-NickelLegierung aufgedampft werden. Solche Katalysatoren sind in der Anmeldung mit dem Titel "Dünnschichtkatalysatoren auf Basis von Raney-Legierungen und Verfahren zu deren Herstellung", Aktenzeichen 199 63 443.2 (Anmelderin: BASF AG) beschrieben.

Gemäß einer unteren bevorzugten Ausführungsform der vorliegenden Erfindung kommen Palladium-Dünnschichtkatalysatoren zum Einsatz, die durch Auftränken dargestellt wurden, vorzugsweise nach dem in der EP-A-827 944 beschriebenen Verfahren.

Dünnschichtkatalysatoren weisen den Vorteil auf, daß sie keinen Abrieb bei einer hohen mechanischen Belastung und eine gute Regenerierbarkeit beim Eintreten von nachlassender Aktivität zeigen.

Die erfindungsgemäße Hydrierung unter Verwendung der oben beschriebenen heterogenen Katalysatoren wird vorzugsweise im Festbett durchgeführt. Dabei hat es sich als vorteilhaft erwiesen, die Reaktion in einem Hauptreaktor im geraden Durchgang durchzuführen. Genauso gute Resultate wurden erhalten, wenn die Hydrierung in einem Hauptreaktor mit nachgeschaltetem Nachreaktor durchgeführt wurde, wobei der Hauptreaktor in Kreislauffahrweise und der Nachreaktor im geraden Durchgang betrieben wurde. Es kann dabei jeweils die Sumpf- oder Rieselfahrweise gewählt werden.

Ebenfalls ist der Einsatz von homogenen Hydrierkatalysatoren möglich. Dabei wird der gelöste Katalysator nach der Reaktion auf die übliche Weise entfernt, beispielsweise in einem nachgeschalteten Verdampfer.

Die erfindungsgemäße Hydrierung des destillativ vorgereinigten TMP kann mit oder ohne Zugabe eines weiteren Lösungsmittels erfolgen. Wird ein solches Lösungsmittel verwendet, so wird dieses in solchen Konzentrationen zugegeben, daß die in die Hydrierung eingesetzten Lösungen einen TMP-Gehalt von 5 bis 95 Gew.-% aufweisen. Als Lösungsmittel werden dabei bevorzugt leichtsiedende organische Lösungsmittel wie Alkohole, Ether, Kohlenwasserstoffe, oder Ester eingesetzt. Bevorzugte Lösungsmittel umfassen Methanol, Ethanol, n-Propanol, i-Propanol, Butanol, Diethylether, Methyl-tert-Butylether, Dioxan, Tetrahydrofuran und Essigsäureethylester. Besonders bevorzugte Lösungsmittel sind Methanol und Tetrahydrofuran. Wird die erfindungsgemäße Hydrierung in Gegenwart eines zusätzlichen Lösungsmittels durchgeführt, so wird hinter die Hydrierung eine Trenneinheit geschaltet, um das Lösungsmittel von dem derart erhaltenen TMP abzutrennen. Übliche Trenneinheiten sind beispielsweise Destillationskolonnen, Dünnschichtverdampfer und vorzugsweise Fallfilmverdampfer.

Das erfindungsgemäße Verfahren wird nun anhand der nachfolgenden Beispiele erläutert. Dabei wurde in allen Beispielen TMP verwendet, das wie folgt hergestellt worden war:

Eine Apparatur bestehend aus zwei beheizbaren, durch Überlaufrohre miteinander verbundenen Rührkesseln mit einem Fassungsvermögen von insgesamt 72 1 wurde mit frischer, wäßriger Formaldehydlösung (4300g/l in Form der 40 %igen wäßrigen Lösung und n-Butyraldehyd (1800 g/h) und mit frischem Trimethylamin als Katalysator (130 g/h) in Form der 45 %igen wäßrigen Lösung kontinuierlich beschickt. Der Reaktoren wurden dabei auf 40°C temperiert.

Der Austrag wurde direkt in den oberen Teil eines Fallfilmverdampfers mit aufgesetzter Kolonne (11 bar Heizdampf) geleitet und dort bei normalem Druck destillativ in ein leichtsiedendes Kopfprodukt, im wesentlichen enthaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45 %igen wäßrigen Lösung) versetzt und in einen beheizbaren, mit Füllkörpern versehenen Rohrreaktor mit einem Leervolumen von 12 1 geführt. Der Reaktor war dabei auf 40°C temperiert.

Der Austrag des Nachreaktors wurde kontinuierlich in den oberen Teil einer weiteren Destillationseinrichtung, der Formaldehydabtrennung (11 bar Heizdampf), gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylolbutyraldehyd. Dieses Sumpfprodukt wurde dann einer kontinuierlichen Hydrierung unterworfen. Dazu wurde die Reaktionslösung bei 90 bar und 115°C in einem Hauptreaktor in Kreislauf/Rieselfahrweise und einem nachgeschalteten Nachreaktor in Kreislauffahrweise hydriert. Der Katalysator wurde analog D der DE 198 09 418 hergestellt. Er enthielt 24 % CuO, 20 % Cu und 46 % TiO₂. Die verwendete Apparatur bestand aus einem 10 m langen beheizten Hauptreaktor (Innendurchmesser: 27 mm) und einem 5,3 m langen beheizten Nachreaktor (Innendurchmesser: 25 mm). Der Kreislaufdurchsatz betrug 25 l/h Flüssigkeit, der Reaktorzulauf wurde auf 4 kg/h eingestellt. Dementsprechend wurden 4 kg/h Hydrieraustrag erhalten.

Das TMP wurde nach der Hydrierung dem Sumpf entnommen und entsprechend der in Beispielen 2 und 3 der deutschen Anmeldung mit dem Titel "Verfahren zur Reinigung von durch Hydrierung hergestelltem Trimethylpropan durch kontinuierliche Destillation", Aktenzeichen 199 63 435.1 (Anmelderin: BASF AG) beschriebenen Methode destillativ aufgearbeitet. In einigen Beispielen wurde dabei TMP verwendet, das der ersten Reindestillationskolonne entnommen wurde (Qualität A). In anderen Beispielen wurde TMP verwendet, das der zweiten Reindestillation entstammte (Qualität B).

Das bereits mit dem erfindungsgemäßen Verfahren hydrierte TMP kann einer weiteren Hydrierung zur Verbesserung der Farbzahl unterworfen werden, wobei vorzugsweise ein anderer Katalysator verwendet wird.

Die angegebenen APHA-Farbzahlen wurden mit einem LICO 200-Gerät der Firma Dr. Lange gemessen. Die TMP-Proben wurden dabei nicht in reiner Form, sondern aufgrund der besseren Reproduzierbarkeit als 50 %-iges Gemisch mit Methanol gemessen. Standardmäßig wurden jeweils 2 Messungen durchgerührt. Der erhaltene Wert wurde anschließend auf eine 100 %ige TMP-Lösung umgerechnet, durch Multiplikation mit dem Faktor 2. Da sich derart nur Farbzahlen bis zu einer Untergrenze von 6 APHA bestimmen lassen, findet sich nachfolgend die Angabe ≤ 6 APHA in den Fällen, in denen ein solcher Wert gemessen wurde.

### Beispiel 1

Es wurde ein TMP der Qualität A hydriert, das eine Farbzahl von 106 Apha und einen TMP-Gehalt von 99,1 % aufwies. Die Hydrierung erfolgte dabei in einem Rohrreaktor in Sumpffahrweise an 130 ml eines Katalysators, der aus 0,5 % Pd auf Al₂O₃ bestand. Die Temperatur betrug 120°C und der Druck 20 bar, es wurde ein Zulauf von 0,85 ml/Min. eingestellt. Nach der Hydrierung erhielt man ein TMP mit einer APHA-Farbzahl von 48.

### Beispiel 2

Ein TMP der Qualität A wurde in einem Rohrreaktor in Sumpffahrweise an 130 ml eines Katalysators, der aus 60 % CuO auf TiO₂ (hergestellt gemäß DE-A-198 09 418) bei einer Temperatur von 100°C und einem Druck von 30 bar hydriert. Der gewählte Zulauf betrug 1,00 ml pro Minute. Das nach der Hydrierung erhaltene TMP wies eine APHA-Farbzahl von 34 auf.

### Beispiel 3

Es wurde verfahren wie in Beispiel 2 beschrieben, wobei die gewählte Temperatur 120°C betrug. Das nach der Hydrierung erhaltene TMP wies eine APHA-Farbzahl von 24 auf.

### Beispiel 4

Es wurde ein TMP der Qualität A verwendet mit einer Farbzahl von 106 APHA und einem TMP-Gehalt von 99,1 %. TMP wurde in Form einer 50 %-igen methanolischen Lösung in Sumpffahrweise an 130 ml eines Cu-TiO₂-Katalysators mit einem Gehalt von 60 % CuO bei einem Druck von 30 bar, einer Temperatur von 140°C und einem Zulauf von 1,0 ml pro Minute hydriert. Das nach der Hydrierung erhaltene Produkt wurde am Rotationsverdampfer von Methanol befreit. Man erhielt ein TMP mit einer APHA-Farbzahl von ≤ 6.

### Beispiel 5

Die Hydrierung wurde durchgeführt wie in Beispiel 4 beschrieben, wobei der Zulauf zu 2,0 ml pro Minute gewählt wurde. Das nach Hydrierung erhaltene Produkt wies eine APHA-Farbzahl von 10 auf.

### Beispiel 6

TMP der Qualität A mit einer Farbzahl von 84 APHA und einem TMP-Gehalt von 99,1 % wurden wie in Beispiel 5 beschrieben hydriert. Der erhaltene Hydrieraustrag wurde bei 120°C und einem Druck von 20 mbar in einem Fallfilmverdampfer von Methanol befreit. Der Sambayablauf enthielt nach gaschromatographischer Analyse 99,1 % TMP und weniger als 0,1 % Methanol. Das teilkondensierte Kopfprodukt enthielt 1,5 % TMP. Es wurde ein TMP mit einer APHA-Farbzahl von 18 erhalten.

### Beispiel 7

TMP der Qualität A mit einer Farbzahl von 64 APHA und einem Gehalt an TMP von 99.1 % wurden wie in Beispiel 5 beschrieben hydriert. Der erhaltene Hydrieraustrag wurde bei 130°C und unter Normaldruck in einem Fallfilmverdampfer mit N₂ als Strippgas bei 10 Nl/h von Methanol befreit. Der Sambayablauf enthielt nach gaschromatographischer Analyse 99,1 % TMP und war frei von Methanol. Im Kopfprodukt wurden 0,7 % TMP festgestellt. Es wurde ein TMP mit einer Farbzahl von 20 APHA hergestellt.

### Beispiele 8 bis 11

Es wurde jeweils ein TMP der Qualität A oder B mit einer Reinheit von 99,1 % verwendet. Die Hydrierung wurde durchgeführt in einer 1,5 1 Rührapparatur mit Begasungsrührer, der bei 1400 min⁻¹ betrieben wurde. Es wurden die in der nachfolgenden Tabelle spezifizierten Dünnschichtkatalysatoren, die nach der EP-A-827 944 hergestellt waren und in monolithischer Form eingebaut wurden, verwendet. Diese wurden durch Tränken auf Metallgewebe bestehend aus dem Werkstoff 1.4767 hergestellt, die anschließend zu einer Packung mit den üblichen Dimensionen gesickt wurden. Es wurden 1300 g geschmolzenes TMP eingebracht und auf die entsprechende Temperatur hochgeheizt, bevor 40 Nl/h Wasserstoff durch das flüssige TMP bei Atmosphärendruck geleitet wurden. Die Ergebnisse finden sich in der folgenden Tabelle.

| Beispiel | Katalysator | mg Metall/m² | Wellmodul [mm] | VWZ [min] | Temp. [°C] | FZ (Edukt) [APHA] | FZ (Produkt) [APHA] | Qualität des verwendeten TMP |
|---|---|---|---|---|---|---|---|---|
| 8 | Pd 1.4767 | 504 | 1,0 | 15 | 120 | 56 | ≤ 6 | B |
| 9 | Pd/Ni 1.4767 | 634 Pd 89 Ni | 1,0 | 15 | 120 | 56 | 12 | B |
| 10 | Pd 1.4767 | 494 | 1,0 | 15 | 120 | 78 | 18 | A |
| 11 | Pd 1.4767 | 286 | 1,0 | 15 | 120 | 78 | 20 | A |
| VWZ = Verweilzeit | | | | | | | | |
| FZ = Farbzahl | | | | | | | | |

### Beispiel 12 bis18

Das als Ausgangsmaterial dienende TMP wies die Qualität B und eine Reinheit von 99, 1 % auf.

In einen 300 ml-Rührautoklav mit Begasungsrührer (1400 U/min) wurde der entsprechende Dünnschichtkatalysator (monolithische Form) eingebracht. Die Pd- und Ru-Dünnschichtkatalysatoren wurden durch Tränkung auf Metallgewebe gemäß EP 0 827 944 hergestellt, welches anschließend zu einer monolithischen Pakkung gewickelt wurde (Wellmodul: 1,5 mm). Nach Einfüllen von 245 g geschmolzenem TMP wurde auf die entsprechende Temperatur hochgeheizt und anschließend der entsprechende Wasserstoffdruck aufgepresst. Nach bestimmten Zeitabständen wurden Proben zur Farbzahlbestimmung entnommen.

| **Beispiel** | **Katalysator** | **mg Metall/m**^{**2**} | **VWZ** | **Temp.** | **Druck** | **FZ (Edukt)** | **FZ (Produkt)** |
|---|---|---|---|---|---|---|---|
| | | | **[min]** | **[°C)** | **[bar]** | **[APHA]** | **[APHA]** |
| 12 | Ru | 578 | 60 | 140 | 30 | 42 | 16 |
| 13 | Ru | 578 | 60 | 140 | 1 | 42 | 34 |
| 14 | Pd+Ru* | 578 | 60 | 140 | 30 | 40 | 14 |
| 15 | Raney-Ni** | 500 | 60 | 140 | 30 | 32 | 14 |
| 16 | Raney-Ni** | 3630 | 60 | 140 | 10 | 38 | 6 |
| 17 | Raney-Ni** | 3630 | 60 | 140 | 1 | 38 | 20 |
| 18 | Raney-Ni** | 3630 | 15 | 140 | 20 | 38 | 8 |

| | | | | | | | |
|---|---|---|---|---|---|---|---|
| * die beiden Ru- und Pd-Dünnschichtkatalysatoren wurden zusammen als Mischung in die Hydrierung eingesetzt. | | | | | | | |
| **Die Raney-Nickel-Katalysatoren wurden gemäß Beispiel 1 der deutschen Patentanmeldung "Dünnschichtkatalysatoren auf Basis von Raney-Legierungen und Verfahren zu deren Herstellung", Aktenzeichen 199 63 443.2 (Anmelderin: BASF AG) erhalten. | | | | | | | |

### Beispiele 19 bis 21

Die Versuche wurden analog Beispiel 18 durchgeführt, wobei jedoch ein Pd auf 1.4767-Gewebe-Dünnschichtkatalysator (504 mg Metall/m², Wellmodul 1,5 mm) eingesetzt wurde. Unter den Bedingungen des Beispiels 12 wurden verschiedene TMP-Qualitäten der Hydrierung unterworfen:

| **Beispiel** | **Einsatzmaterial** | **TMP-Gehalt** | **FZ (Edukt)** | **FZ (Produkt)** |
|---|---|---|---|---|
| 19 | TMP (Qualität B) | 99,0 % | 38 | 16 |
| 20 | Entwässertes Roh-TMP* | 83 % | > 600 | > 600 |
| 21 | Entwässertes + von Hochsiedem befreites Roh-TMP** | 94 % | > 600 | > 600 |

| | | | | |
|---|---|---|---|---|
| * entwässert gemäß der deutschen Anmeldung "Farbzahlverbesserung von mehrwertigen Alkoholen durch kontinuierliche Destillation", Aktenzeichen 199 63 435.1 (Anmelderin: BASF AG), entnommen nach der Wasser- und Leichtsiederabtrennung | | | | |
| ** entwässert gemäß der deutschen Anmeldung "Farbzahlverbesserung vonmehrwertigen Alkoholen durch kontinuierliche Destillation", Aktenzeichen 199 63 435.1 (Anmelderin: BASF AG), entnommen nach der Wasser- und Leichtsiederabtrennung und der Abtrennung der Hochsieder | | | | |

Dieses Beispiel zeigt, daß sich nur destillativ vorgereinigte TMP-Qualitäten eignen, durch Hydrierung in der Farbzahl verbessert zu werden.

### Beispiel 22

Es wurde ein TMP der Qualität A mit einer Farbzahl von 120 APHA unter den Bedingungen des Beispiels 16 hydriert, wobei diese Hydrierung bei 30 bar (statt 10 bar) durchgeführt wurde. Das nach Hydrierung erhaltene TMP wies eine Farbzahl von 18 APHA auf.

### Beispiele 23 bis 24

Das als Ausgangsmaterial dienende TMP der Qualität B wies eine Farbzahl von 56 APHA sowie eine Reinheit von 99,1 % auf. Die Hydrierung wurde in einem Rohrreaktor, der mit 8 Einsätzen an gewickeltem Pd-Kanthal-Dünnschichtkatalysator gefüllt war, durchgerührt. Die TMP-Lösung wurde in Sumpf- oder Rieselfahrweise über die Katalysatorschüttung geleitet und ein Teil des TMP-Reaktionsaustrags wurde in den Reaktor zurückgeführt (Kreislauffahrweise). Die Wasserstoffmenge betrug 20-40 l/h.

Nach Erreichen des stationären Zustands wurden Proben zur Farbzahlbestimmung entnommen.

| **Beispiel** | **Sumpf/Riesel** | **Kreislaufmenge** | **H2- menge** | **Zulauf** | **VWZ** | **Temp.** | **FZ (Produkt)** |
|---|---|---|---|---|---|---|---|
| | | **[l/h]** | **[l/h]** | **[ml/min]** | **[min]** | **[°C)** | **[APHA)** |
| 23 | S | 20 | 20 | 2,0 | 60 | 120 | 18 |
| 24 | R | 12 | 20 | 2,0 | 60 | 120 | 18 |

### Beispiel 25

Der Versuch wurde analog Beispiel 24 durchgeführt, jedoch wurde als Edukt TMP der Qualität B mit der Farbzahl 16 APHA eingesetzt, die Kreislaufmenge betrug 20 l/h, die H₂-Menge ebenfalls 60 l/h. Durch die Hydrierung wurde die Farbzahl auf ≤ 6 APHA erniedrigt.

## Patentansprüche

1. Verfahren zur Farbzahlverbesserung von mehrwertigen Alkoholen aus der Gruppe bestehend aus Trimethylolethan, Trimethylolpropan, Trimethylolbutan, Neopentylgylkol und Pentaerythrit, insbesondere Trimethylolpropan, durch katalytische Hydrierung, **dadurch gekennzeichnet, daß** der in der Hydrierung eingesetzte mehrwertige Alkohol nach seiner Herstellung durch Destillation gereinigt wurde.

2. Verfahren nach Anspruch 1, **dadurch gekennzeichnet, daß** der mehrwertige Alkohol aus dem anorganischen oder dem organischen Cannizzaro-Verfahren oder dem Hydrierverfahren, vorzugsweise dem Hydrierverfahren, stammt.

3. Verfahren nach Anspruch 1 oder 2, **dadurch gekennzeichnet, daß** der in der Hydrierung eingesetzte mehrwertige Alkohol einen Produktgehalt > 95 %, vorzugsweise, im Fall von Trimethylolpropan, > 98 % aufweist.

4. Verfahren nach einem der Ansprüche 1 bis 3, **dadurch gekennzeichnet, daß** heterogene Katalysatoren von Metallen der 3. bis 12. Gruppe des Periodensystems, vorzugsweise Cu, Ni, Pd, Ru sowie Kombinationen dieser Metalle, sowohl in Form der reinen Metalle als auch von deren Verbindungen, in der Hydrierung benutzt werden.

5. Verfahren nach Anspruch 4, **dadurch gekennzeichnet, daß** auf Trägern, vorzugsweise. Al₂O₃ oder TiO₂, aufgebrachte Katalysatoren in jeglichen Konfektionierungsformen, vorzugsweise Strängen oder Tabletten, oder Dünnschichtkatalysatoren auf organischen oder anorganischen Materialien oder auf Metallen verwendet werden.

6. Verfahren nach Anspruch 5, **dadurch gekennzeichnet, daß** Dünnschichtkatalysatoren mit Pd, Ru oder Raney-Nickel in sämtlichen möglichen Konfektionierungen des Gewebes verwendet werden.

7. Verfahren nach einem der Ansprüche 1 bis 6, **dadurch gekennzeichnet, daß** die Hydrierung bei Temperaturen von 20 bis 300°C, vorzugsweise 100 bis 180°C, und Drücken von 1 bis 350 bar, vorzugsweise 1 bis 100 bar, meist bevorzugt 1 bis 50 bar, durchgeführt wird.

8. Verfahren nach einem der Ansprüche 1 bis 7, **dadurch gekennzeichnet, daß** die Verweilzeit des mehrwertigen Alkohols von 5 min bis 4 Stunden, vorzugsweise 10 min bis 60 min, beträgt.

9. Verfahren nach einem der Ansprüche 1 bis 8, **dadurch gekennzeichnet, daß** die Hydrierung diskontinuierlich, vorzugsweise im Rührkessel, oder kontinuierlich, vorzugsweise in Rohrreaktoren in Sumpf- oder Rieselfahrweise, betrieben wird.

## Claims

1. A process for improving the color index of polyhydric alcohols from the group consisting of trimethylolethane, trimethylolpropane, trimethylolbutane, neopentyl glycol and pentaerythritol, especially trimethylolpropane, by catalytic hydrogenation, wherein the polyhydric alcohol used in the hydrogenation has been purified by distillation following its preparation.

2. A process as claimed in claim 1 wherein the polyhydric alcohol originates from the inorganic or organic Cannizzaro process or from the hydrogenation process, preferably from the hydrogenation process.

3. A process as claimed in claim 1 or 2 wherein the polyhydric alcohol used in the hydrogenation has a product content of >95% or, in the case of trimethylolpropane, preferably >98%.

4. A process as claimed in any of claims 1 to 3 wherein heterogeneous catalysts of metals of groups 3 to 12 of the periodic table, preferably Cu, Ni, Pd or Ru, as well as combinations of these metals, either in the form of the pure metals or in the form of compounds thereof, are used in the hydrogenation.

5. A process as claimed in claim 4 wherein catalysts applied to supports, preferably Al₂O₃ or TiO₂, in any form, preferably in the form of rods or tablets, or film catalysts on organic or inorganic materials or on metals, are used.

6. A process as claimed in claim 5 wherein film catalysts containing Pd, Ru or Raney nickel are used in any of the possible forms of the fabric.

7. A process as claimed in any of claims 1 to 6 wherein the hydrogenation is carried out at temperatures of 20 to 300°C, preferably 100 to 180°C, and pressures of 1 to 350 bar, preferably 1 to 100 bar and particularly preferably 1 to 50 bar.

8. A process as claimed in any of claims 1 to 7 wherein the residence time of the polyhydric alcohol is from 5 minutes to 4 hours, preferably 10 minutes to 60 minutes.

9. A process as claimed in any of claims 1 to 8 wherein the hydrogenation is carried out batchwise, preferably in a stirred tank, or continuously, preferably in tubular reactors by the liquid phase or trickle method.

## Revendications

1. Procédé d'amélioration d'indice de couleur d'alcools polyvalents du groupe constitué de triméthyloléthane, de triméthylolpropane, de triméthylolbutane, de néopentylglycol et de pentaérythrite, en particulier de triméthylolpropane, par hydrogénation catalytique, **caractérisé en ce que** l'alcool polyvalent mis en oeuvre dans l'hydrogénation a été purifié par distillation après sa préparation.

2. Procédé suivant la revendication 1, **caractérisé en ce que** l'alcool polyvalent est issu du procédé de Cannizzaro inorganique ou organique ou du procédé d'hydrogénation, de préférence du procédé d'hydrogénation.

3. Procédé suivant la revendication 1 ou 2, **caractérisé en ce que** l'alcool polyvalent mis en oeuvre dans l'hydrogénation présente une teneur en produit > 95%, de préférence, dans le cas de triméthylolpropane, > 98%.

4. Procédé suivant l'une des revendications 1 à 3, **caractérisé en ce qu'**on utilise dans l'hydrogénation des catalyseurs hétérogènes de métaux des groupes 3 à 12 du système périodique, de préférence Cu, Ni, Pd, Ru, ainsi que des combinaisons de ces métaux, aussi bien sous la forme des métaux purs que de leurs composés.

5. Procédé suivant la revendication 4, **caractérisé en ce qu'**on utilise des catalyseurs appliqués sur des supports, de préférence Al₂O₃ ou TiO₂, sous des formes de confection quelconques, de préférence d'extrudés ou de comprimés, ou des catalyseurs en couche mince sur des matières organiques ou inorganiques ou sur des métaux.

6. Procédé suivant la revendication 5, **caractérisé en ce qu'**on utilise des catalyseurs en couche mince comportant du Pd, du Ru ou du nickel de Raney dans l'ensemble des confections possibles du tissu.

7. Procédé suivant l'une des revendications 1 à 6, **caractérisé en ce que** l'hydrogénation est effectuée à des températures de 20 à 300°C, de préférence de 100 à 180°C, et à des pressions de 1 à 350 bars, de préférence de 1 à 100 bars, très avantageusement de 1 à 50 bars.

8. Procédé suivant l'une des revendications 1 à 7, **caractérisé en ce que** le temps de séjour de l'alcool polyvalent est de 5 minutes à 4 heures, de préférence de 10 minutes à 60 minutes.

9. Procédé suivant l'une des revendications 1 à 8, **caractérisé en ce que** l'hydrogénation est effectuée de manière discontinue, de préférence dans une cuve d'agitation, ou de manière continue, de préférence dans des réacteurs tubulaires dans un mode de fonctionnement en fond de cuve ou à ruissellement.
